# EUROPEAN PATENT APPLICATION

(11) **EP 2 871 230 A1**
(43) Date of publication of application: **13.05.2015**
(21) Application number: 13813972.0
(22) Date of filing: 01.07.2013
(51) Int. Cl.: C12M 1/00

(54) **MICROCHAMBER CHIP FOR CELL SPREADING**

(30) Priority: 03.07.2012 JP 2012149407
(71) Applicant: Konica Minolta, Inc., Tokyo 100-7015 (JP); National Institute Of Advanced Industrial Science, Tokyo 100-8921 (JP)
(72) Inventor: HOSHI, Kumiko, Tokyo 100-7015 (JP); ARAKI, Jungo, Tokyo 100-7015 (JP); YAMAMURA, Shohei, Takamatsu-shi Kagawa 761-0395 (JP); YATSUSHIRO, Shouki, Takamatsu-shi Kagawa 761-0395 (JP); KATAOKA, Masatoshi, Takamatsu-shi Kagawa 761-0395 (JP)
(74) Representative: Gille Hrabal
(86) International application number: PCT/JP2013/067980
(87) International publication number: WO 2014/007192

(57) **Abstract**

Provided is a micro chamber chip for cell expansion, with which the non-specific adsorption of cells onto a surface other than a micro chamber can be suppressed, and a rare cell from a substance that contains a large quantity of cells, such as a blood, can be stored, held and observed without a leakage of the rare cell. A micro chamber chip for cell expansion is provided with a micro chamber chip (20) in which a micro chamber (30) configured to store and hold one or more cells is formed on an upper surface (11) of a substrate (10), wherein an upper surface (11) of the micro chamber chip (20) and an inner wall surface (31) of the micro chamber (30) are coated with a blocking agent (50) that can suppress a non-specific adsorption of a cell on the upper surface (11).

## Description

### [Technical Field]

The present invention relates to a micro chamber chip for cell expansion in which a blocking treatment is applied to an upper surface of the micro chamber chip and the inner wall surface of a micro chamber.

### [Background Art]

A circulating tumor cell (CTC), a vascular endothelial cell, an endothelial progenitor cell, a variety of stem cells and the like (referred to as "a rare cell" collectively in this specification) are cells present very rarely in the whole blood depending on pathological conditions. Despite obvious clinical usefulness of the rare cells, the detection of the rare cells is very difficult. The detection of the rare cell has been attempted by applying a variety of cell separation techniques and the commercialization has been made in recent years. However, because of the scarcity of the target, the evaluation of the effectiveness of the detection result (loss of rare cells or the presence/absence of the incorporation of unwanted cells) is important.

In the examination of the presence/absence of a targeted rare cell in a specimen material such as blood drawn, for example, a cell suspension such as a blood-derived specimen material is planarly expanded and then the whole cell expanded is analyzed to know whether or not there is a targeted cell in the cell suspension.

Where the cell suspension is planarly expanded using a plane which is for example a chemical micro device disclosed in Patent Literature 1, cells can be stored in fine recesses formed on the device surface (micro wells or micro chambers) at a high density and the cells can be detected for each recess. Further, it is possible to collect a cell from each recess one by one and to subject the cell to further testing. The chemical micro device disclosed in Patent Literature 1 can be easily manufactured by injection molding using a plastic material which generates less fluorescence by the excitation light. However, when the cell is expanded by using this chemical micro device, since a cell is easily adsorbed on other surfaces in addition to the recesses, there is a possibility that the cells expanded cannot be collected sufficiently from the recesses, causing the loss of rare cells.

On the other hand, Patent Literature 2 discloses a biochip with fine holes that can fix individually target materials one by one. In the biochip disclosed in Patent Literature 2, the same or different immobilization materials may be linked onto both side-surfaces (walls) and bottom-surfaces (bottoms) of the fine holes, or the immobilization materials may be linked to either the side-surfaces or the bottom-surfaces. Further, Patent Literature 2 discloses that, for example when a target material is a "lymphocyte cell that expresses the antigen recognition region corresponding", "a variety of antibodies" can be selected as an immobilization material.

The biochip disclosed in Patent Literature 2, for example, can immobilize cells of the same kind one by one into the micro holes one by one, but is not provided in order to detect the rare cells from e. g. , blood made of an aggregation composed of extremely large number of cells of different types and sizes. Even when an immobilization material corresponding to a rare cell (for instance, antibodies to antigens expressed specifically on the surface of the rare cell) is linked to either the side-surfaces or the bottom-surfaces, or both, of the fine holes, the immobilization material cannot capture the rare cells once the fine holes are filled with cells other than the rare cells. In addition, a suitable immobilization material corresponding to a targeted rare cell does not always exist. Here comes the advantage of a method in which cells are expanded onto a micro chamber chip for cell expansion whereby almost all cells are stored in the micro chamber and rare cells are detected by the observation using a microscope.

### [Citation List]

### [Patent Literature]

[Patent Literature 1]
   JP-A- No. 2004-212048
[Patent Literature 2]
   JP-A- No. 2005-214889

### [Summary of Invention]

### [Problems to be Solved by the Invention]

An object of the present invention is to provide a micro chamber chip for cell expansion, with which the non-specific adsorption of cells onto a surface other than a micro chamber can be suppressed, and a rare cell from a substance containing a large quantity of cells, such as a blood, can be stored, held, and observed without leakage of the rare cell.

### [Means for Solving the Problems]

The present inventors have found the following and completed the present invention. By the surfaces of the micro chamber chip and part or whole of the inner wall surfaces of the micro chamber that are coated with a blocking agent capable of suppressing non-specific adsorption of the cells, when cells of many types in a large amount contained in the blood or the like are developed on the chip, the cells are easily stored and held in the micro chamber and further the cells are hardly adsorbed on the inner wall surface of the micro chamber, whereby the cells are not multi-layered with each other in the micro chamber and the observation using a microscope can be carried out easily.

In order to achieve at least one of the purposes described above, the micro chamber chips (41) and (42) for cell expansion in accordance with the present invention are provided with a micro chamber chip (20) in which a micro chamber (30) configured to store and hold one or more cells is formed on an upper surface (11) of a substrate (10), wherein an upper surface (11) of the micro chamber chip (20) and an inner wall surface (31) of the micro chamber (30) are coated with a blocking agent (50) that can suppress a non-specific adsorption of a cell on the upper surface (11).

### [Advantageous Effects of Invention]

When the micro chamber chips (41) and (42) for cell expansion in accordance with the present invention is used for cell expansion, almost every cell can be stored in the micro chamber (30).

The micro chamber chips (41) and (42) for cell expansion have undergone the application of the cell non-adhesive surface treatment (referred to as a "blocking treatment") extended to the inner wall surface of the micro chamber (30), whereby cells easily gather on the bottom surface without being adsorbed on the inner wall of the micro chamber (30), which can facilitate the bright-field observation of cells under a microscope.

Furthermore, since a blocking treatment is not applied to the bottom surface of the micro chamber (30), to which cells easily adsorb, in the present invention, the cells that have gathered on the bottom surface of the micro chamber (30) hardly escape through a subsequent solution sending operation, so that it is possible to prevent a loss of rare cells without using an immobilization material such as an antibody that recognizes an antigen on the cell surface, thereby improving the reliability of the cell observation.

Furthermore, the micro chamber chips (41) and (42) for cell expansion in accordance with the present invention can be produced by easily applying a blocking treatment both to the upper surface (11) of the substrate (10) having the micro chamber (30) formed thereon, i.e., the micro chamber chip (20), and to the inner wall surface (31) of the micro chamber (30). That is to say, the micro chamber chips (41) and (42) for cell expansion in accordance with the present invention can be produced in only one step of a blocking treatment to surfaces excluding the bottom surface (32) of the micro chamber (30) of the micro chamber chip (20).

### [Brief Description of the Drawings]

[Fig. 1]

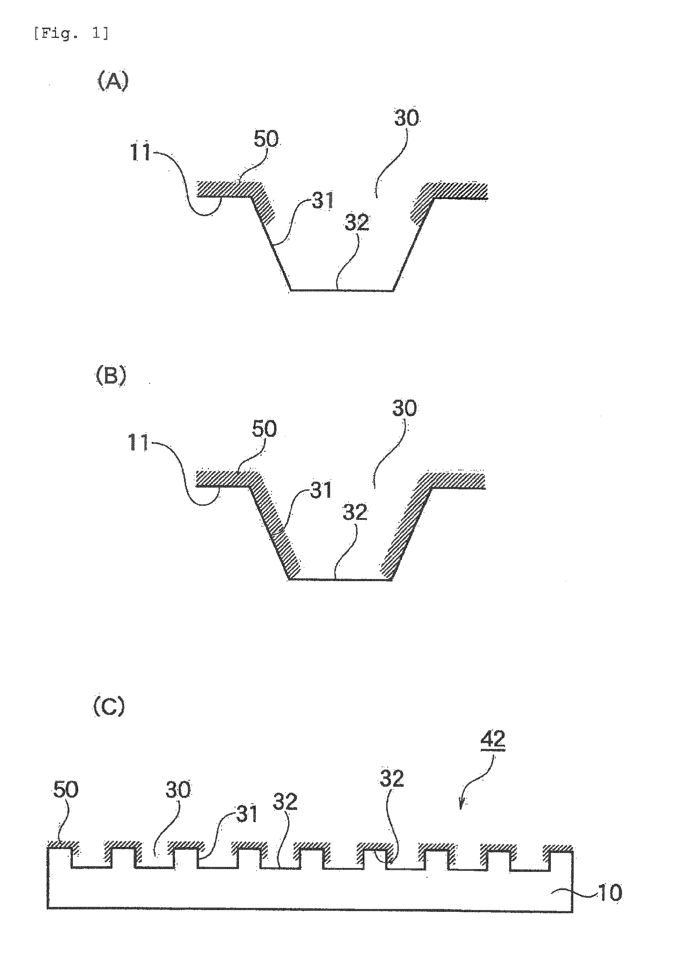

   Figs. 1(A) and 1(B) are views schematically showing a vertical cross sectional view of a typical micro chamber (30) included in the micro chamber chip for cell expansion in accordance with the present invention.
   Fig. 1(C) is a view schematically showing a vertical cross sectional view of the micro chamber chip (42) for cell expansion that is obtained by a mode of a method for producing the micro chamber chip for cell expansion in accordance with the present invention, and shows the way part or whole of the inner wall surface (31) of the micro chamber (30) is coated with a blocking agent (50).
[Fig. 2]

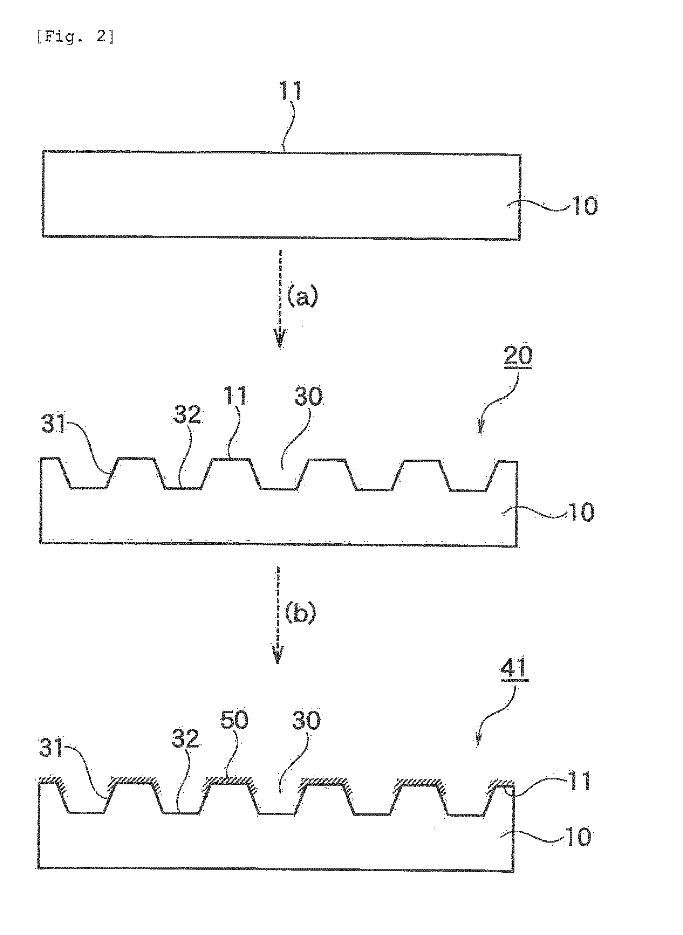

   Fig. 2 is a view schematically showing steps (a) and (b) in one mode of the method for producing a typical micro chamber chip (41) for cell expansion in accordance with the present invention.
[Fig. 3]

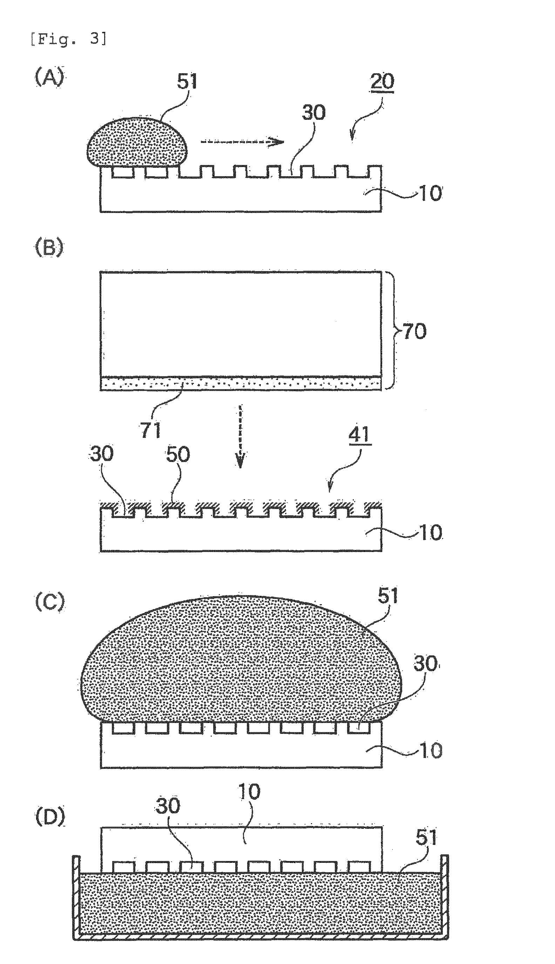

   Fig. 3(A) is a view schematically showing (b-1) of the step (b) in one mode of the method for producing a typical micro chamber chip for cell expansion in accordance with the present invention;
   Fig. 3(B) is a view schematically showing (b-2) of the step (b) in one mode of the producing method;
   Fig. 3(C) is a view schematically showing (b-3) of the step (b) in one mode of the producing method; and
   Fig. 3(D) is a view schematically showing (b-4) of the step (b) in one mode of the producing method.
      An arrow in Fig. 3(A) indicates the direction for sending a blocking treatment solution (51), and an arrow in Fig. 3(B) indicates the direction for pressing a stamp (70) to the micro chamber chip (20). A blocking treatment solution (51) has penetrated into an inkpad (71) of Fig. 3(B).
[Fig. 4]

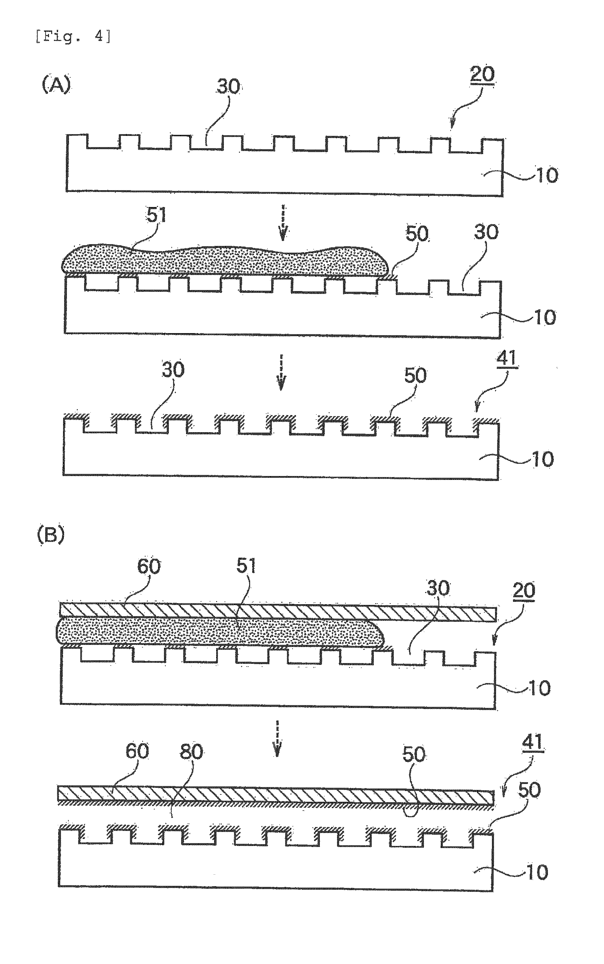

   Fig. 4(A) is a view giving an explanation in more detail for Fig. 3(A), and Fig. 4(B) is a view showing a mode in which a top plate (60) is disposed above the micro chamber chip (20) to form a flow path (80) for Fig. 3(A).
[Fig. 5]

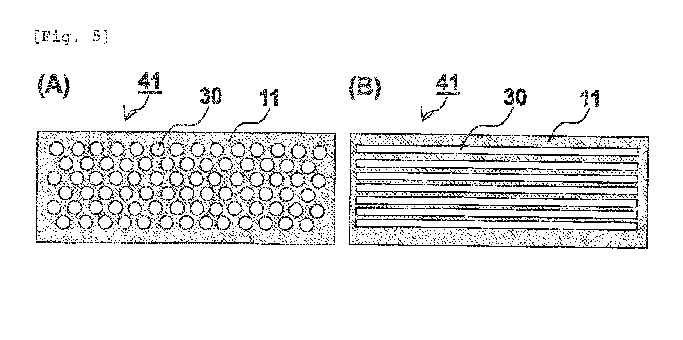

   Fig. 5 is a schematic diagram of the micro chamber chip (41) for cell expansion in accordance with the present invention seen from up above, which indicates a case (A) in which an opening part of the micro chamber (30) is in a circular shape and a case (B) in which an opening part of the micro chamber (30) is in a rectangular shape.
[Fig. 6]

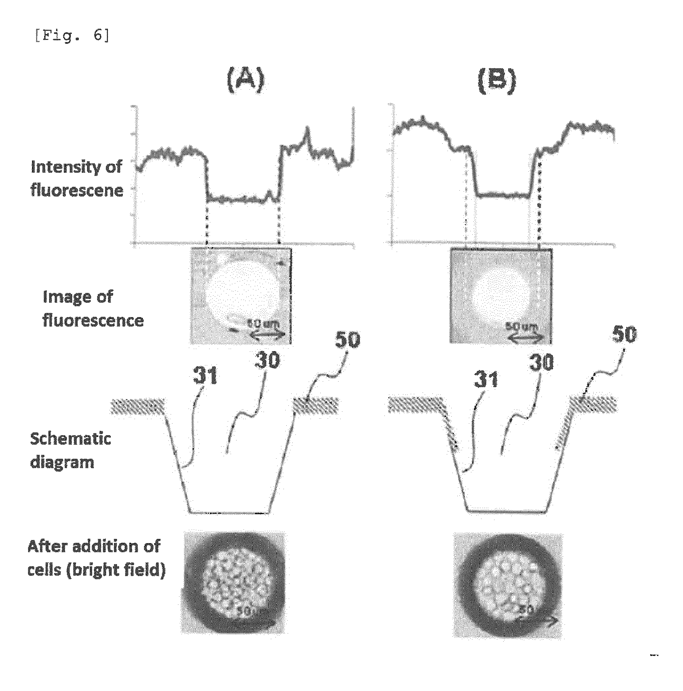

   Figs. 6(A) and 6(B) show the results of Example 1.

### [Description of Embodiments]

The present invention will be described in detail in the following.

### <Micro chamber chip for cell expansion>

According to Figs. 1 and 2, the micro chamber chips (41) and (42) for cell expansion in accordance with the present invention are characterized by comprising a micro chamber chip (20) in which a micro chamber (30) configured to store and hold one or more cells is formed on an upper surface (11) of a substrate (10), wherein the upper surface (11) of the micro chamber chip (20) and an inner wall surface (31) of the micro chamber (30) are coated with a blocking agent (50) that can suppress a non-specific adsorption of a cell on the upper surface (11). It is preferable that the application of the blocking agent (50) is continuous from the upper surface (11) of the substrate (10) to the inner wall surface (31) of the micro chamber (30). However, an actual mode includes a micro chamber (30) non-continuously coated with no blocking agents applied on a boundary between the upper surface (11) and the inner wall surface (31). The present invention includes such a mode, too. The inner wall surface (31) indicates parts (side surface) excluding the bottom surface (32) in surfaces that configure the micro chamber (30).

When the micro chamber included in the micro chamber chip for cell expansion in accordance with the present invention is enlarged, the present invention includes, for example: a mode in which part (that is, an edge) of the inner wall surface (31) of the micro chamber (30), in addition to the upper surface (11) of the substrate (10), is coated with the blocking agent (50) as shown in Fig. 1(A); and a mode in which the entirety of the inner wall surface (31) is coated with the blocking agent (50) as shown in Fig. 1(B). In other words, the entirety from the opening part toward the bottom part in the inner wall surface (31) of the micro chamber (30) may be coated with the blocking agent (50) (Fig. 1(B)), and part from the opening part to a halfway toward the bottom part may be partially coated with the blocking agent (50) (Fig. 1 (A)). On the other hand, the bottom surface (32) is prevented from being coated with the blocking agent (50) so that the cells stored inside the micro chamber (30) once do not escape by the subsequent solution sending.

In the micro chamber included in the micro chamber chip (42) for cell expansion shown in Fig. 1(C), the micro chamber (30) includes both the modes of Fig. 1 (A) and Fig. 1(B). The micro chamber included in the micro chamber chip (41) for cell expansion shown in Fig. 2, wherein the micro chamber (30) is configured only in accordance with the mode of Fig. 1 (A), can be said to be a typical example.

By the blocking treatment being applied to the inner wall surface (31) of the micro chamber (30) in addition to the upper surface (11), the cells expanded can be prevented from being adsorbed on the inner wall surface (31) of the micro chamber (30), in particular on the edge, whereby the cells held on the edge and the bottom surface (32) of the micro chamber (30) do not overlap with each other, thereby enabling a suitable observation of the cells under a microscope.

### <Manufacturing method of the micro chamber chip for cell expansion>

As shown in Fig. 2 for example, it is preferable that a method of manufacturing the micro chamber chip (41) for cell expansion in accordance with the present invention includes steps (a) and (b) that will be described below.

### [Micro chamber forming step (a)]

The step (a) is a process for producing a micro chamber chip (20) by forming the micro chamber (30) on the upper surface (11) of the substrate (10).

The method of forming the micro chamber is not limited particularly. A method known in the art may be used. There can be mentioned for example a method of molding using a metal mold with a projection corresponding to a shape of the micro chamber, a method of the injection molding using a silicone resin, and a method of forming a micro chamber by applying a direct processing (such as a fine processing by lithography, a drilling process, and a LIGA process) to a substrate that is made of a polymer such as a thermoplastic resin, a metal, or a glass. In terms of an industrial mass production, a method of molding using a metal mold is preferable.

### (Substrate)

A material of the substrate used in the present invention can be the same material as a micro plate or the like conventionally known or can be a material that can be formed using a mold. There can be mentioned for example polymers such as polystyrene, polyethylene, polypropylene, polyamide, polycarbonate, polydimethyl siloxane [PDMS], polymethyl methacrylate [PMMA], and a cyclic olefin copolymer [COC] as the material. The substrate may be a combination of a plurality of materials such as a material made by bonding a substrate made of metal, glass, quartz glass to a (metal-molded) polymer.

When a contact angle between water and the substrate is measured by dropping water on the substrate by using a dynamic contact angle meter ("FTA125" manufactured by FTA Co., Ltd.), the contact angle is in the range of 20 degrees to 80 degrees preferably, 30 degrees to 70 degrees more preferably. The contact angle of 20 degrees or larger indicates that the hydrophilicity of the substrate intended to be brought into contact with a blocking treatment solution (an aqueous solution in many cases) described later is not so high. Consequently, the contact angle of 20 degrees or larger is preferable since the blocking treatment solution (51) can be prevented from entering excessively inside the micro chamber (30) to coat the bottom surface (32) in the blocking treatment step (b) in accordance with the present invention.

### (Micro chamber)

The micro chamber in accordance with the present invention means a minute hole in a concave shape (a micro well) that is able to "store" and "hold" one cell or more. Here, the term "store" means that a cell enters (is housed in) a micro chamber when the cell suspension is added to the surface of the micro chamber chip for cell expansion in accordance with the present invention, and the term "hold" means that the cell stored in the micro chamber does not get out of the micro chamber by a stain solution or a cleaning solution added to the surface of the micro chamber chip for cell expansion.

When the opening part of the micro chamber (30) is in a circular shape, its diameter can be about 500 µm. Since the cell-retaining force of the micro chamber (30) is particularly strong, it is preferable that the diameter is in the range of 20 µm to 150 µm. When the opening part of the micro chamber (30) is in a shape other than the circular shape, it is preferable that the dimensions of the shape are equivalent to those of the circle in the above range.

It is preferable that the depth of the micro chamber (30) is varied according to the diameter of the micro chamber (30). A person having ordinary skill in the art can determine appropriately the depth of the micro chamber in such a manner that cells of 10 to 15 pieces can be stored for every chamber. It is preferable that the depth of the micro chamber (30) is in the range of 20 µm to 100 µm.

By adjusting a ratio between the diameter or the dimensions of the opening part of the micro chamber (30) and the depth of the micro chamber (30) to the appropriate extent, the blocking treatment solution is allowed to maintain a sufficient surface tension and can be prevented from reaching the bottom surface (32) of the micro chamber (30).

The shape of the opening part (horizontal cross section) of the micro chamber (30) is circular typically as shown in Fig. 5 (A). However, the opening part may also be in a rectangular shape as shown in Fig. 5(B) and is not particularly limited in terms of shape.

The shape of the micro chamber (30) is typically a reverse circular truncated cone shape (a vertical cross section shows a trapezoidal shape as shown in Fig. 1(A) and 1(B)) or a cylindrical shape (a vertical cross section shows a rectangular shape as shown in Fig. 3). However, the present invention is not restricted to the mode. It is preferable that the dimensions of the opening part of the micro chamber (30) are larger than or equivalent to those of the bottom surface (32). As the shape of the micro chamber (30), there can be mentioned for instance a reverse hemispherical shape, a reverse pyramid shape (a reverse polygon cone shape such as a reverse square cone and a reverse hexagonal pyramid), a cuboid shape in addition to the reverse circular truncated cone shape and the cylindrical shape.

As long as the micro chamber (30) can hold a cell, the micro chamber (30) may not be provided with a bottom part (a cell is held at the side wall around the bottom part) or may have a bottom partially having a hole for the purpose of eliminating a solvent of the cell suspension. However, it is preferable that the micro chamber (30) is provided with the bottom part (that is, not a through hole) from the viewpoint of decreasing as much as possible the risk of a loss of rare cells. Although the bottom surface (32) of the micro chamber (30) is typically planar, it may also be curved.

### (Micro chamber chip)

The micro chamber chip (20) obtained in the step (a) is the one in which the micro chamber (30) is formed on the upper surface (11) of the substrate (10). In the present invention, since the upper surface (11) of the substrate (10) is coated with a blocking agent in the blocking treatment step (b) carried out next, it is not necessary that a processing for achieving a blocking effect (in particular, a processing that could lead to coat the bottom surface with a blocking agent) is carried out in the step (a) in which the micro chamber (30) is formed. If necessary, the surface treatment in a range in which the effects of the present invention is not inhibited, for instance a surface treatment to enhance the cell adhesion of the substrate such as a UV ozone treatment, may be carried out before the blocking treatment.

### [Blocking treatment step (b)]

The step (b) is a step for bringing the blocking treatment solution (51) including a blocking agent (50) into contact with the upper surface (11) of the micro chamber chip (20) obtained in the step (a) to coat the upper surface (11) of the substrate (10) and (part or whole of) the inner wall surface of the micro chamber (30) with the blocking agent (50).

As specific examples of embodiments of the blocking treatment step (b), there can be mentioned for example four methods (b-1) to (b-4) that will be described in the following.

### (Blocking agent / blocking treatment solution)

The blocking agent (50) refers to a substance that by coating the upper surface (11) of the substrate (10) suppresses cells from being non-specifically adsorbed thereto. Further, the blocking treatment solution (51) refers to a solution that is used in the blocking treatment step (b) of the present invention and that is prepared by diluting the blocking agent (50) with a suitable solvent. The blocking treatment solution (51) includes the blocking agent (50) (for instance, a mode of dissolution or dispersion is included).

It is possible to use known materials as the blocking agent. There can be mentioned for example a hydrophilic polymer such as casein, skimmilk, albumin (including bovine serum albumin [BSA]), and polyethylene glycol, phospholipids, and low molecular compounds such as ethylene diamine and acetonitrile. One kind can be used alone or the combination of at least two kinds can also be used.

The solvent for diluting the blocking agent may be properly selected depending on the blocking agent. For example, when BSA is used as a blocking agent, a solvent preferred is the one which is adapted into a biologically-relevant substance that is similar to a solvent in which cells to be expanded are suspended. There can be mentioned for example a phosphate buffered saline [PBS], HEPES, MEM, RPMI, and a phosphate buffer solution.

### [Flow velocity control method (b-1)]

The method (b-1) is also called a flow velocity control method, and is a method for sending the blocking treatment solution (51) (such as a BSA solution) from one end of the upper surface (11) of the substrate (10), as shown in Fig. 3(A) and Fig. 4(A).

As a more preferred embodiment of the flow velocity control method, as shown in Fig. 4 (B), there can be mentioned for example a method in which a top plate (60) is formed on the upper surface (11) side of the substrate (10) in such a manner that the top plate (60) is parallel to the micro chamber chip (20) to form a "flow path" (80) and the blocking treatment solution (51) is sent from one end of the flow path. In the present specification, such an embodiment is referred to also as "in-flow-path flow velocity control method". The in-flow-path flow velocity control method is preferable since each operation of the expansion, dyeing and detection of cells can be carried out in a closed system subsequent to the blocking treatment step (b).

For the in-flow-path flow velocity control method, it is preferable that the distance between the upper surface (11) of the micro chamber chip (20) and the inner surface of the top plate (60) (hereinafter referred to as "height of the ceiling") is in the range of 50 µm to 1000 µm. By the micro chamber chip (20) and the top plate (60) being separate from each other by such a distance, moderate capillary force works to expand the blocking treatment solution in full scale, thereby applying the blocking treatment solution not only to the substrate but also to the substrate-side surface of the top plate (60) at the same time, and the cell suspension can also be expanded to the micro chamber chip (20) in full scale and can be stored in the micro chamber (30).

In the flow velocity control method, a means to flow down the blocking treatment solution (51) is not particularly limited. For example, it is preferable that the blocking treatment solution (51) (and the cell suspension) is sent into the closed system by using a solution sending means such as a solution sending pump for the in-flow-path flow velocity control method. When the closed system is not used, the micro chamber chip (20) can be inclined or the blocking treatment solution can be sucked by using a suction means (a water-absorbing member such as filter paper for example) from the downstream side.

It is preferable that a speed of the solution sending (a flow velocity) of the blocking treatment solution is in the range of 0.1 to 1000 mm/sec. By the flow velocity being in this range, the blocking treatment solution can easily be prevented from excessively entering the micro chamber (30) to apply a blocking treatment to the bottom surface (32), too. When the contact angle between water (the blocking treatment solution is an aqueous solution in many cases) and the substrate (10) is greater than 20 degrees, it is also possible that a flow velocity of the solution sending can be adjusted in a range less than 1 mm/s. In the in-flow-path flow velocity control method, a flow velocity at the surface of the substrate and a flow velocity at the center part of the flow path (the midpoint of the top plate and the upper surface of the substrate) are different from each other in some cases. However, in the present invention, a flow velocity of the solution sending of the blocking treatment solution approximately corresponds to a flow velocity at the center part of the flow path. On the other hand, a flow rate is of a pump for the solution sending. For example, in the case of using a flow path with a width of 5 mm and a height of the ceiling of 100 µm, a flow velocity in the range of 0.1 to 1000 mm/s corresponds to a flow rate in the range of 0.003 to 30 mL/min.

Furthermore, it is preferable that a flow velocity of the blocking treatment solution (51) is adjusted so as to ensure a contact time during which the substrate (10) can be sufficiently coated with the blocking agent (50) by a width along the flow-down direction of the region in which the micro chamber (30) is formed. It is preferable that the contact time in this case is adjusted in the range of 10 seconds to 1 hour. By making the contact time relatively short, the blocking agent (50) can be applied from the upper surface (11) of the substrate (10) to the middle of the inner wall surface (31) of the micro chamber (30) as shown in Fig. 1(A). By making the contact time relatively long, the blocking agent can be applied to the entirety of the inner wall surface (31) of the micro chamber (30) as shown in Fig. 1(B). If the contact time is excessively long, the bottom surface (32) of the micro chamber (30) can be also coated with the blocking agent (50). On the other hand, if the contact time is excessively short, the blocking agent may not adhere well to the upper surface (11) and the inner wall surface (31), degrading the blocking effect.

### [Stamp method (b-2)]

The method (b-2) is also referred to as a stamp method, in which a "stamp" (70) with an "inkpad" (71) impregnated with the blocking treatment solution (51) is brought into contact with (or is pressed to) the upper surface (11) of the substrate (10) as shown in Fig. 3(B). This method is primarily applied to a micro chamber chip (20) of an open system not provided with a top plate (60). The stamp method (b-2) can enable a suitable blocking treatment to a micro chamber chip (20) to which a blocking treatment is hard to apply with the flow velocity control method (b-1), the simple addition method (b-3) or the simple immersion method (b-4).

As a manufacturing method of the stamp (70), there can be mentioned for example a method of forming an inkpad (71) by applying the UV ozone treatment to a smooth surface of a silicone rubber made of polydimethyl siloxane [PDMS] to modify the surface to be hydrophilic and immersing the inkpad in the blocking treatment solution (51) such as PBS in which BSA has been dissolved.

The treatment conditions for pressing the stamp (70) to the upper surface (11) of the substrate (10) can be adjusted in an appropriate range in such a manner that the blocking treatment solution (51) used for impregnating the inkpad (71) does not excessively enter the micro chamber (30) to apply a blocking treatment to the bottom surface (32). Although the pressure of a suitable stamp pressing may vary depending on a material of the stamp, when a stamp (or an inkpad) of a silicone rubber made of PDMS as described above is used, it is preferable that the pressure of stamp pressing is adjusted in the range up to e.g., 100 MPa/cm². Although the time of stamp pressing may vary depending on a pressure of stamp pressing, the time can be appropriately adjusted in a range similar to the contact time for the flow rate control method (b-1) as described above.

### [Simple addition method (b-3)]

The method (B-3) is also referred to as a simple addition method, in which the blocking treatment solution (51) is dropped over the whole of the upper surface (11) of the substrate (10) as shown in Fig. 3 (C). This method is primarily applied to a micro chamber chip (20) of an open system not provided with a top plate (60). Although the upper surface (11) of the substrate (10) is coated with the blocking treatment solution (51) dropped, the surface tension of the blocking treatment solution (51) prevents the blocking treatment solution (51) from excessively entering the micro chamber (30). When the blocking treatment solution (51) is an aqueous solution, the contact angle between water and the substrate can be mentioned as a control of the degree of entering the micro chamber (30). When the contact angle is smaller, the degree of entering of the blocking treatment solution (51) becomes higher. If the contact angle is, for example, 100 degrees, the blocking treatment is applied substantially to the upper surface alone of the substrate. However, when the contact angle is 80 degrees or less, the blocking treatment solution (51) starts to enter the micro chamber (30) so that the application of the blocking treatment is extended to part of the inner wall surface (31) of the micro chamber (30). In order to prevent the blocking treatment solution (51) from entering the bottom surface (32) of the micro chamber (30), it is preferable that the contact angle is in the range of 20 degrees to 80 degrees, more preferably in the range of 30 degrees to 70 degrees.

In this method, the time during which the application of the blocking treatment solution (51) is kept from the upper surface (11) of the substrate (10) to the inner wall surface (31) of the micro chamber (30) can be appropriately adjusted in a range similar to the contact time for the flow velocity control system (b-1) as described above. After the above treatment, the blocking treatment solution that dropped may be removed from the substrate (10).

### [Simple immersion method (b-4)]

The method (b-4) is also referred to as a simple immersion method, in which the micro chamber chip (20) is turned upside down and the whole of the upper surface (11) of the substrate (10) is immersed in the blocking treatment solution (51) as shown in Fig. 3(D). When this method is applied to a micro chamber chip (20) of a closed system provided with a top plate (60), one surface of the top plate (60) or the entirety of the top plate (60) may be immersed in the blocking treatment solution (51) before disposing the top plate (60).

In this method, the time during which the micro chamber chip (20) turned upside down is immersed in the blocking treatment solution (51) can be appropriately adjusted in a range similar to the contact time for the flow velocity control system (b-1) as described above. When the depth for immersion is larger, the blocking treatment solution (51) enters the micro chamber (30) farther. However, because of the surface tension of the blocking treatment solution (51) and bubbles present in the micro chamber (30), the blocking treatment solution (51) is prevented from coming into contact with the bottom surface (32) of the micro chamber (30). A desired depth for immersion is varied depending on the depth or the shape of the micro chamber (30) itself. However, a person having ordinary skill in the art can adjust the depth for immersion appropriately.

### [Example]

While embodiments in accordance with the present invention will be described in detail in the following, the present invention is not restricted to the embodiments.

### [Example 1]

### <Preparation of a cell suspension>

The PBS solution (1 × 10⁷ cells/mL) of Jurkat cells was prepared as a cell suspension.

### <Micro chamber chip>

A micro chamber chip (length × width is 25 mm × 70 mm) was prepared from the substrate made of polystyrene by using a predetermined metal mold. A diameter of the opening part of the micro chamber was 100 µm, a depth of the micro chamber was 50 µm, and the micro chamber was provided with a flat bottom surface and was in a reverse conical shape.

Furthermore, the UV ozone treatment for 1 minute was carried out by using the UV ozone cleaner manufactured by Meiwa Forsyth Co., Ltd. and the contact angle of the micro chamber chip and water was measured by the dynamic contact angle meter (FTA105) of manufactured by FTA Co., Ltd. As a result, the contact angle was 70 degrees.

### <Blocking treatment>

First, a smooth surface of a silicone rubber made of PDMS was modified to be hydrophilic by the UV ozone treatment for 60 minutes ("PC440" manufactured by Meiwa Forsyth Co., Ltd.), and then the smooth surface of a silicone rubber was immersed into PBS that contains Alexa Fluor (registered trademark) 488 conjugate (manufactured by Invitrogen Co., Ltd.) of BSA by 0.5% by weight for 1 hour, to prepare a stamp.

This stamp was pressed to the micro chamber chip for 20 minutes at 0.1 MPa/cm².

Thereafter, the surface of the micro chamber chip was washed with PBS, thereby obtaining a micro chamber chip for cell expansion in which the blocking agent (50) was coated to the middle of the inner wall surface (31) of the micro chamber (30) as shown by the schematic diagram of Fig. 6(B).

The He-Ne laser (a wavelength of 633 nm) that excites the Alexa Fluor (registered trademark) 488 labeled for the BSA labeling was applied, and the fluorescence intensity of BSA that had coated the micro chamber chip for cell expansion was measured by using the photomultiplier tube [PMT]. Fig. 6 (B) shows a graph in which the fluorescence intensity was plotted on the horizontal axis of the maximum width of the opening part of one micro chamber (that is, a width corresponding to the diameter).

### <Cell expansion>

First, a top plate was disposed to the micro chamber chip for cell expansion obtained, and the upper surface of the micro chamber chip for cell expansion and the inner surface of the top plate were separated from each other by 100 µm. In addition, a side wall was formed in such a manner that a width of the flow path is 5 mm. Thereafter, PBS of 10 mL, 70% ethanol of 1 mL, and PBS of 10 mL were sent in this order at a flow velocity of 1 mm/sec.

In such a manner that the PBS introduced in the flow path would be replaced, a cell suspension of 200 µL prepared in advance was introduced to the flow path at a flow velocity of 1 mm/sec, and was allowed to be left for 5 minutes to settle the cells. Thereafter, an extra liquid component (a cell suspension from which most cells had been removed) was discharged from the flow path.

The "intermittent solution sending", in which the PBS was introduced from the flow path for 0.1 seconds at a flow velocity of 1 mm/sec and was allowed to be left for 10 seconds, was repeated 20 times.
Fig. 6 (B) shows an image taken in bright-field observation under a microscope.

### [Comparative example 1]

The fluorescence intensity derived from BSA was measured in the same manner as in Example 1 except for changing the time for pressing the stamp to the micro chamber chip from 20 minutes to 10 seconds in the <blocking treatment> of Example 1. Thereafter, the cell expansion was carried out, and cells were observed by a bright field of a microscope and imaged. The results are shown in Fig. 6(A).

### (Consideration)

When the stamp time is 10 seconds (Comparative example 1: Fig. 6(A)), cells are adsorbed also to the inner wall surface of the micro chamber and overlap with cells lying on the bottom surface, so that it can be difficult to observe the cells. On the other hand, when the stamp time is 20 minutes (Example 1: Fig. 6(B)), since the blocking treatment has been applied also to the inner wall surface of the micro chamber, the cells gather at the bottom surface, thereby facilitating the observation.

### [Reference Signs List]

- 10:: Substrate
- 11:: Upper surface
- 20:: Micro chamber chip
- 30:: Micro chamber
- 31:: Inner wall surface of the micro chamber (30)
- 32:: Bottom surface of the micro chamber (30)
- 41 and 42:: Micro chamber chips for cell expansion
- 50:: Blocking agent
- 51:: Blocking treatment solution
- 60:: Top plate
- 70:: Stamp
- 71:: Inkpad
- 80:: Flow path

## Claims

1. A micro chamber chip for cell expansion, comprising a micro chamber chip (20) in which a micro chamber (30) configured to store and hold one or more cells is formed on an upper surface (11) of a substrate (10), wherein the upper surface (11) of the micro chamber chip (20) and an inner wall surface (31) of the micro chamber (30) are coated with a blocking agent (50) that can suppress a non-specific adsorption of a cell on the upper surface (11).

2. The micro chamber chip for cell expansion as defined in claim 1, wherein a diameter of an opening part of the micro chamber (30) is in the range of 20 µm to 150 µm and a depth of the micro chamber (30) is in the range of 20 µm to 100 µm.

3. The micro chamber chip for cell expansion as defined in claim 1 or 2, wherein the micro chamber (30) is bottomed.

4. The micro chamber chip for cell expansion as defined in any one of claims 1 to 3, wherein the blocking agent (50) includes an at least one type that is selected from the group consisting of casein, skim milk, albumin, a hydrophilic polymer molecule and phospholipid.

5. The micro chamber chip for cell expansion as defined in any one of claims 1 to 4, wherein a contact angle between water and the substrate (10) is in the range of 20 degrees to 80 degrees.
